# EUROPEAN PATENT SPECIFICATION

(11) **EP 1 240 514 B1**
(45) Date of publication and mention of the grant of the patent: **12.08.2009**
(21) Application number: 00985625.3
(22) Date of filing: 20.12.2000
(51) Int. Cl.: G01N 33/50

(54) **USE OF A BIOLOGICAL SENSOR FOR SENSING ANTIBIOTICS**
VERWENDUNG EINES BIOLOGISCHEN SENSORS ZUM NACHWEIS VON ANTIBIOTIKA
UTILISATION D'UN CAPTEUR BIOLOGIQUE POUR DETECTER D'ANTIBIOTIQUES

(30) Priority: 20.12.1999 GB 9929892
(43) Date of publication of application: 18.09.2002
(73) Proprietor: QinetiQ Limited, London, SW1E 6PD (GB)
(72) Inventor: PARSONS, Alan, Thomas, Dorchester, Dorset DT2 8XJ (GB); HEAL, Richard, David, Albert, Dorchester, Dorset DT2 8XJ (GB)
(74) Representative: Knight, Samuel
(86) International application number: PCT/GB2000/004881
(87) International publication number: WO 2001/046456

(56) References cited:
- EP-A- 0 773 292
- US-A- 5 759 846
- GROSS GUENTER W ET AL: "Odor, drug and toxin analysis with neuronal networks in vitro: Extracellular array recording of network responses." BIOSENSORS & BIOELECTRONICS, vol. 12, no. 5, 1997, pages 373-393, XP001009727 ISSN: 0956-5663 cited in the application
- GROSS GUENTER W ET AL: "The use of neuronal networks on multielectrode arrays as biosensors." BIOSENSORS & BIOELECTRONICS, vol. 10, no. 6-7, 1995, pages 553-567, XP001009730 ISSN: 0956-5663 cited in the application

## Description

This invention relates to biological sensors. More particularly the present invention relates to the use of living neuronal cells for sensing an antibiotic.

It is known that biological materials exist in nature that employ micro-systems capable of performing physical sensing functions. The benefit of employing such materials is that the materials are likely to be sensitive, efficient, abundant and adaptable. More importantly, when using live materials it is likely that they will work better since the systems would, in nature, be of little use to a dead organism.

Problems exist in the identification and isolation of the appropriate organisms, and with the harnessing of their outputs in a repeatable and coherent manner.

It is already known, for example, that neurons exist which can perform many of these basic sensing functions. It is also known, from early work in Japan (FUKADA E, YASUDA I. On the piezoelectric effect of bone. J. Phys. Soc. of Japan. 12, 1158-1162.FUKADA E (1968). Piezoelectricity in polymers and biological materials. Ultrasonics. 68, 229-234.FUKADA E, ANDO Y. (1988). Bending piezoelectricity in a microbially produced poly-b-hydroxybutyrate. Biorheology 297-302) that dried bone possesses piezo-electric properties.

Further work has been performed more recently at the "Centre Technique des Systèmes Navals" (CTSN) Toulon in conjunction with the University of Marseilles under funding from the French government. In the more recent work the investigations have included vegetable protein structures (cellulose), fungal material, algae and yeasts, all of which have been found to possess interesting sensory properties.

US 5,759,846 discloses a system for permitting the long term survival of mammal CNS slice cultures in order to study the electrophysiological activity and biochemical analysis of cerebral parenchyma. The system comprises, *inter alia,* three dimensionally organized tissue and extracellular recording electrodes. With the system, continuous and simultaneous stimulation and recording of neuronal activity can be performed during several days.

EP 0773292 discloses the generation of stable immortalised neural cell lines and clones derived therefrom. The cells represent a useful tool for screening compounds with potential neuroprotective activity.

The sensors of the present invention use neurological cell cultures. The advantage of using neuronal cells in a biological acoustic sensor is that they are likely to be already endowed with very sensitive pressure sensing systems and have an inherent reporter system. The problem is in the detection and interpretation of a suitable response from a population of such cells.

*In vivo* measurements of auditory evoked potentials have long been routine (e.g. Urbani and Lucertini (1994), Hearing Res. 76:73) and studies on individual isolated neurones, such as dorsal root ganglion cells, were made possible by voltage- or current-clamping techniques (Christenson et al (1993) Brain Res. 608: 58). Measurement of some physiological responses to, for instance, oxygen, carbon dioxide and ammonia levels, had been possible since at least the 1960s by means of microelectrode sensing from single neurones (Negishi and Svaetichin (1966) Pflueg Arch 292: 177).

The present inventors sought to develop suitable electrophysiology equipment for the monitoring of neuronal activity and to identify and culture potential pressure sensitive neurons.

The present inventors have prepared useful neuronal cells and cell lines together with specialised electrophysiological equipment. Initial investigations of the spontaneous output from neuronal cells have been made.

Advantageously, the growth conditions of spontaneously active neuronal networks on the specialised equipment have been optimised. Modifications were made to the experimental set up to enable pressure stimuli to be applied.

The present inventors have found that it is in fact possible to produce electrically active dorsal root ganglia (DRG) cultures. Previously, it has been shown that active rat DRG cultures may be produced from excised tissue but electrical activity has only been analysed by intracellular techniques. Such studies have shown oscillation of the membrane potential and periodic firing [AMIR R, DEVOR M Spike-evoked suppression and burst patterning in dorsal root ganglion neurons of the rat. J. Physiol. (London) 501, 183-196.] The results of the present inventors constitute the first documented case of extracellular recording of electrical activity oscillations from DRG cultures. Such findings have important implications as the DRG cells are beginning to be analysed in terms of pain and nerve injury.

Pressure sensing apparatus may comprise living neuronal cells contacting a substrate, wherein said substrate comprises a plurality of electrodes capable of detecting the electrical activity of said cells.

Preferably, said neuronal cells are primary dorsal root ganglion cells.

Alternatively, said neuronal cells are selected from the group comprising ND7/23 cells, neuromast cells, P-cells, stretch receptor cells, nodose cells, Merckel cells, phaeochromocytoma cells, Immortomouse cells and hair cells.

The pressure sensing apparatus may comprise a diaphragm capable of transmitting an external acoustic signal to the fluid medium contacting said neuronal cells.

The system may be used in a drug screening method not claimed. Surprisingly, the present inventors have found that the addition of antibiotics to the cell culture medium has an acute and chronic effect on neuronal activity. This effect has not previously been documented in the literature.

According to the invention, there is provided the use of apparatus comprising living neuronal cells contacting a substrate, wherein said substrate comprises a plurality of electrodes capable of detecting the electrical activity of said cells, for sensing an antibiotic, **characterised in that** said antibiotic comprises an aminoglycoside or polymixin 'B'.

Embodiments of the invention will now be described in more detail with reference to the following drawings of which;
Figure 1 shows a mature culture of foetal rat hippocampal neurons;
Figure 2 shows an example of a trace recording of spontaneous activity from a 4-week old culture of hippocampal neurons;
Figure 3 shows three waveforms characteristic of spontaneous activity in hippocampal neuronal cultures (a) is classical action potential profile, (b) is a sodium spike and (c) is a rare chloride spike;
Figure 4 shows spontaneous activity recorded from mature cultures of foetal rat spinal cord neurons;
Figure 5 shows a culture of primary dorsal root ganglia cells from foetal rats;
Figure 6 is a representative trace of spontaneous activity recorded from mature cultures of primary foetal rat dorsal root ganglia cells;
Figure 7 shows a characteristic waveform of spontaneous events recorded from a 4-week old culture of dorsal root ganglia cells;
Figure 8 shows the effect of the acute addition of antibiotics to the spontaneous activity of hippocampal neuronal cell cultures, (a) Penicillin G, (b) Streptomycin, (c) Penicillin G/ Streptomycin, (d) Kanamycin (arrow shows point of addition);
Figure 9 shows the modifications to the MMEP system to enable application of static pressures to neuronal cells;
Figure 10 shows the modification of the burst intervals in DRG spontaneous activity in response to an applied static pressure, (a) is a representative trace with pressure applied at the arrow and (b) is a graph indicating burst interval timings between bursts;
Figure 11 is a trace showing the stimulation of DRG burst activity in response to a drop of medium falling 10 cm onto the culture;
Figure 12 shows the bioacoustic calibration tube;
Figure 13 shows the MMEP base plate and culture chamber modifications to allow pressure application to neuronal cultures present in the bioacoustics calibration tube;
Figure 14 is a trace showing spontaneous activity from foetal rat spinal cord neurons present inside the bioacoustics calibration tube, and
Figure 15 is a recording obtained from a 2 week old differentiated culture of the ND7/23 (dorsal root ganglia) cell line where a pressure of 1 atm was applied transiently at point P1 and the trace was switched off at point O for approximately 3 minutes before the pressure was re-applied at point P2.

### Example 1 : Neuronal Biological Sensor

Experiments have been performed investigating the effects of a wide range of antibiotic compounds on neuronal network activity in cultured hippocampal cells.

Preliminary pressure response experiments were performed on active networks of dissociated primary dorsal root ganglia cells. The results showed an alteration of neuronal activity in response to an applied pressure stimulus.
Experiments have also been performed attempting to generate neuronal networks from cell lines. Two cell lines have been tested and, the cells readily differentiate into a neuronal-like morphology.

Nerve cell culture: typically nerve cells are cultured by two methods, tissue slices and dissociated cell culture. Nerve tissue slices represent the most highly organised neuronal cultures and are no more than thin slices of the nerve tissue of interest. Neuronal tissue slices are typically maintained for study for up to 6 hours. Dissociated neuronal cultures, on the other hand, are prepared by total dispersion of the neural tissue into single cells by mechanical and enzymatic methods. The cells are then seeded at an appropriate density in tissue culture-treated plasticware containing nutrient medium and incubated at 37°C. Neuronal cells usually require additional coating of the plastic surface to improve attachment, prevent clumping, and stimulate the growth of processes. Over a period of a few days functional connections (synapses) are established between cells and within approximately seven days the cells reach full maturity. Mature neuronal cultures have been reported to survive for up to several months [ BANKER G, GOSLIN K (1991) Culturing nerve cells. MIT Press].

Dissociated cell preparations are referred to as 'primary' cultures because they are derived directly from living tissue. Cell lines, however, usually originate from a tumour extracted from an animal or human and can be allowed to multiply up to potentially unlimited quantities in appropriate conditions. A neuronal example is the Phaeochromocytoma (PC12) cell line originally derived from a tumour of a rat adrenal gland. The PC12 line multiplies under standard tissue culture conditions as clumps of small, round, non-adherent cells. Additional coating of the tissue culture plasticware, and the inclusion of Nerve Growth Factor (NGF) in the culture medium produces cell cultures with true neuronal morphology [GREENE L A, TISCHLER A S (1976) Establishment of a noradrenergic clonal line of rat adrenal pheochromocytoma cells which respond to nerve growth factor. Proc. Natl.Acad. Sci. USA 73, 2424-2428.].

Cell lines can also be produced artificially by chemical or viral 'immortalisation' or fusion of cells with an already immortal cell line. A relatively recent innovation involves the development of the 'Immortomouse' [HOLLEY M C, LAWLOR P W (1997) Production of conditionally immortalised cell lines from a transgenic mouse. Audiol. Neurootol. 2, 25-35.]. Dissociated cells from tissue from the 'Immortomouse' remains immortalised at 33°C. Transfer of these cells to the normal body temperature of the mouse (39°C) stops cell division and they develop their normal characteristics.

Electrophysiological techniques: the classical method for measuring electrical phenomena associated with neuronal cells involves the insertion of ultrafine glass microelectrodes, filled with electrolyte solution, into isolated or cultured mammalian nerve cells. Recordings of this type indicate that neuronal cells have a resting membrane potential of ( -70mV (the inside being negatively charged). An important advantage of a system of this type, that is, a fixed-array, multi microelectrode system, is that it is non-invasive.

Changes in electrical potential with different characteristics and magnitude are recorded as a nerve impulse travels along an axon, depending on the positioning of the electrodes. In the case of a single electrode, in contact with the surface of a nerve cell, and another at a remote location, i.e. in the bathing medium, there is an initial rise in potential followed by a return to normality, a decrease in potential and a final return to resting conditions. The changes are representative of an initial alteration in the permeability of the membrane to Na+ and K+ ions and subsequent cross-membrane fluxes.

The main disadvantage of the electrodes mentioned above is the need for them to be manipulated into close contact with the neurons of interest. This results in an inherently unstable and short-lived preparation. Recent advances have been made in the use of fixed-array, multi-microelectrode systems. A number of systems have been developed and designed for long-term monitoring of extracellular single unit neuronal activity in vitro. Such systems have been used to monitor spontaneous and electrically or drug induced activity in monolayer cultures of neuronal cells [GROSS G W, WEN W Y, LIN J W (1985). Transparent indium-tin oxide electrode patterns for extracellular, multisite recording in neuronal cultures. J. Neurosci. Meths. 15, 243-252. GROSS G W, SCHWALM F U (1994). A closed flow chamber for long-term multichannel recording and optical monitoring. J. Neurosci. Meths. 52, 73-85. GROSS G W (1994). Internal dynamics of randomised mammalian neuronal networks in culture. In Enabling Technologies for Cultured Neural Networks 13, pp 277-317. Academic Press. GROSS G W, RHOADES B K, AZZAZY H M E, WU M-C (1995) The use of neuronal networks on multielectrode arrays as biosensors. Biosensors & Bio-electronics 10, 553-567.]. Sophisticated electronics have been developed in parallel for the acquisition and analysis of large-scale multichannel activity data [ABUZAID M A, VITHALANI P V, GOSNEY W M, HOWARD L L, GROSS G W (1991). A VLSI peripheral system for monitoring and stimulating action potentials of cultured neurons. Proc. of the 1 st Great Lakes Symp. on VLSI. Kalamazoo, MI, pp. 170-175.].

Mechanoreceptor cells: an exhaustive search was performed to identify potential cells that could be screened, in conjunction with the acquired electrophysiological equipment, for production of signals in response to mechanical stimulation. A report of cultured cells having been used in this manner was not identified. It was, however, demonstrated that a single leech mechanosensory neuron (P cell) did not exhibit spontaneous activity when placed on a linear electrode array, although responses could be evoked by electrical or mechanical stimulation.
Potentially useful cell types identified are listed below.
- Neuromast cells: Isolated from the 'lateral line' of fish and 'stitches' distributed over the body of amphibia. Neuromast cells are a complex conglomeration of different phenotypes, with the 'hair' cell as the basic sensory unit. There are no reports of successful long-term culture of these cells.
- 'P' Cells: Isolated from the leech ventral nerve cord. Although they have been examined for responses to mechanical and electrical stimulation on microelectrode arrays they are not conducive to large scale or long term culture conditions.
- Stretch receptor cells: Isolated from crayfish, they have been used in patch-clamp electrophysiological experiments to study the characteristics of single-channel currents. There are no reports of these cells in tissue culture studies.
- Nodose sensory neurons: Isolated from 'nodose' ganglia of mammals that lie just exterior to the cranial cavity in the upper neck region. The 'nodose' ganglia contain the cell bodies of nodose sensory neurons that innervated the major organs of the body. Nodose sensory neurons are bipolar neurons that are stimulated by distension of the cell membrane. Standard procedures exist for the culture of these neurons and they have been successfully maintained for up to 12 days in vitro [SHARMA RV, CHAPLEAU M W, HAJDUCZOK G, WACHTEL R E, WAITE L J, BHALLA R C, ABBOUD F M (1995). Mechanical stimulation increases intracellular calcium concentration in nodose sensory neurons. Neurosci. 66, 433-441.] by which time well-developed neurites are formed.
- Merckel cells: These are mammalian mechanoreceptor cells that transmit pressure signals from the skin to the primary sensory nerve fibres. A pure monolayer culture of Merckel cells has been reported, and a tumour cell line of human origin is available, although no electrophysiological studies have yet been performed.
   'Hair' cells: Two different types of cells are involved in the transduction of mechanical stimuli in the cochlea of the mammalian ear. The inner hair cells (IHCs) and the outer hair cells (OHCs). Isolated hair cells have been cultured but only very small populations of sensory neurons are obtained. Several lines from the inner ear of the 'Immortomouse' have been isolated and are currently in the process of characterisation.
   Neuronal cell lines: A number of cell lines of neuronal origin are commercially available. Although most are not from source tissue that is mechanoreceptive by nature, the possibility exists that certain surface receptors may be deformed and hence activated by mechanical stress. One potential mechanoreceptive cell line is the ND7/23 cell line. The progenitor tissue of this cell line is the dorsal root ganglion of the mouse.

### Example 2

### Electrophysiological Equipment

In order to produce a neuronal biological sensor of acoustic pressure a robust system for measuring neuronal outputs is required. A system that enables neuronal activity to be measured using extracellular substratum electrodes was identified. This system had only recently become feasible due to advances in neuronal cell culture and sophisticated amplification technologies. It has the advantages of being capable of measuring electrical responses from a population of living cells growing on the substratum, in a non-invasive way, over a protracted period of time.

Miniature microelectrode plate (MMEP) system. This system that has been developed over the last 20 years by Guenter Gross, CNNS, Denton, Texas, USA (Gross *et al* (1997) Biosensors and Bioelectronics 12: 373-393) comprises of three basic components:
a metal base plate fitted with a central recess to accept the microelectrode plates and an optical port allowing microscopic examination of neuronal cells. The plate is fitted with power resistors to enable heating of the cultures to occur;
a microelectrode plate consisting of a 2 inch square indium tin oxide (ITO) - sputtered glass slide which has 64 central terminals (four rows, 16 columns with 200 and 40µm spacing respectively) in an area of 0.8mm². The plates are coated with a 2µm thick layer of insulating polysiloxane resin that is removed over the central terminals by laser pulses. The ITO thus exposed is plated with a thin layer of gold to complete the electrode;
a stainless steel culture chamber comprising a central circular aperture and sealing ring that is clamped over the microelectrode plate.

Electrophysiology amplification equipment. Studies presented herein been performed using the 'Neurolog' system supplied by Digitimer Ltd., Welwyn Garden City, Hertfordshire. A single channel A.C. recording arrangement consists of an impedance buffer headstage unit connected to a preamplifier unit capable of up to 20k gain. The signal is further processed through a filter unit and a gated amplitude discriminator. The signal is displayed visually on an oscilloscope or as sound through an audio amplifier unit. Optimal settings of 10K gain and a filter range of 500Hz to 6kHz are used routinely (as recommended by Gross et al. *supra).*

Multichannel Neuronal Amplifier. The major limitation of using the Neurolog system is that only one channel can be monitored at any one time. Need of multichannel equipment was revealed.

The neuronal amplifier system consists of two 32-channel pre-amplifier boards that are connected to a 64-channel amplifier card. Initial trials using MMEP plates and culture media indicate that the system is capable of providing a gain of 100,000 and has low noise characteristics (background noise 17 nanovolts / (Hz). Using the system neuronal outputs from cultures present inside the BioAcoustic Tube have been recorded.
Real time data acquisition software. Software has been developed to enable the real time analysis and recording of multiple channels of neuronal output data. All 64 channels are interfaced to a dedicated PC with 16, user defined, channels displayed in real time. These channels can be flagged to indicate the presence of activity and the data logged to disk. Saved data can be replayed for analysis or transferred to other software packages for presentation.

Cell Culture Methodology

Detailed methods for the preparation and maintenance of primary cell cultures and cell lines have been described comprehensively in the literature and are well known to the person skilled in the art.

Primary cell culture. The tissue of interest is dissected from a freshly terminated animal in a laminar airflow hood under sterile conditions. For neuronal tissue from the Central Nervous System (CNS) younger animals, in particular embryonic, will yield the best cultures. Tissue is minced, subjected to enzymatic digestion, and titurated to produce a single cell suspension. Cell density is determined and cells are dispersed (seeded) into tissue culture plasticware containing an appropriate volume of medium.

The cultures are held at 37°C in an incubator with an atmosphere of 5% CO2 in air which interacts with the bicarbonate buffer of the medium to maintain pH at a neutral 7.3.

Hippocampal / spinal cord / dorsal root ganglia cells. Hippocampal cultures are produced according to well-documented methods. The dissection of both spinal cord and Dorsal Root Ganglia (DRG) can be performed from the same E15 day foetuses. The head is removed and skin peeled away from the back of the foetus. The spinal cord is then loosened from the spinal canal by teasing with fine watchmaker's forceps, and finally stripped out from the head end down. DRG can be carefully plucked from the cord, the meninges can then be peeled away and the cord isolated. Enzymatic digestion and cell suspension methods are essentially the same as for hippocampal cultures, except DNAse I at .05% is usually included in spinal cord digests. A range of seeding densities has been tested between 50,000 and 200,000 per cm² . In this manner, electrically active DRG cultures can be prepared.

Cell line maintenance. 'Immortal' cells can be cultured over long periods of time and expanded up to copious quantities. Routine maintenance involves removing the cell layer by mechanical or enzymatic means and sub-culturing them into fresh medium at a lower cell density. Some cell lines require coating factors to be present on the tissue cultureware (e.g. PC-12) and many require the presence of specific factors to be present to enable a morphological change to occur. This change is referred to a differentiation and is typified by a stop on multiplication and a change in the characteristics of the cell.

Tissue culture ware. All tissue culture plasticware used was as previous [1]. The use of Heraeus FlexipermTM tissue culture wells has enabled the production of good quality neuronal cell cultures. They are particularly useful for seeding very small areas of the MMEP plates. This is especially useful for producing dorsal root ganglia cultures as only small numbers of these cells are generated in any one dissection run.

Culture medium. Basic medium formulations are available from a variety of commercial sources. The basic formulation is generally supplemented with glutamine, antibiotics and serum to ensure healthy cultures. Media formulations used in this project are:
Hippocampal; spinal cord; DRG cell medium:
   Seeding out: Neurobasal medium (Gibco) + B27 supplement + L-Glutamine (2mM) + 10% Foetal bovine serum (FBS)
   Culture media: Neurobasal medium (Gibco) + B27 supplement + L-Glutamine (2mM)
ND7/23 media formulations:
   Routine culture: DMEM + 10% FBS + L-Glutamine (2mM)
   Differentiation: DMEM + 0.5% FBS + L-Glutamine (2mM) + dibutryl cAMP (1 mM) + 2(g/ml Nerve growth factor (NGF)
P19 cell line:
   Routine culture: MEM + 10% FBS + L-Glutamine (2mM) + Non-essential Amino acids (1x)
   Differentiation: MEM + 0.1 % FBS + L-Glutamine (2mM) + Non-essential Amino acids (1x) + 30uM retinoic acid
   Neurobasal + B27 + Non-essential Amino acids (1x) + L-Glutamine (2mM) + 30uM retinoic acid
PC12 culture medium:
   Routine culture: RPMI 1640 basal medium + 5% FBS + 5% Horse serum + L-glutamine (2mM) + Penicillin (100IU/ml) + Streptomycin (0.1mg/ml)
   Differentiation: As routine medium with addition of NGF (100ng/ml)

### Merckel cell line medium:

Routine culture: MEM with Earles salts + 10% FBS + L-Glutamine (2mM) + Non-essential Amino acids (1x) + Penicillin (100IU/ml) + Streptomycin (0.1mg/ml)
Fish/amphibia medium:
   Routine culture: Leibovitz L-15 + D-glucose (10mM) + Gentamycin 50(g/ml + 5% FBS

### Primary cells and cell lines investigated and acquired

Rat Hippocampal neurons: Cultures of these cells from the learning/memory centre of the brain (embryonic rat) are prepared routinely and some have been utilised in initial calibration of the MMEP system and antibiotic experiments.

Rat Spinal cord cells: Rat foetal spinal cord cells are prepared from E15 day foetus by standard procedures and have been used in initial calibration experiments.

Rat Dorsal Root Ganglia cells. Rat dorsal root ganglia cells are prepared from dissected ganglia by standard techniques. Neuronal cells from the DRG innervate regions of the skin and are, therefore, potentially mechanosensory. ND7/23 cell line: The ND7/23 dorsal root ganglia cell line was acquired from the European Collection of Animal Cell Cultures, Salisbury, UK. This cell line is cultured routinely using standard techniques and has the potential of being mechanosensory.

P19 cell line: The P19 cell line was acquired from the European Collection of Animal Cell Cultures, Salisbury, UK. This cell line is cultured routinely using standard techniques.

PC12 cell line: This cell line is cultured routinely and has been the main source of neuronal cultures for the initial calibration of the MMEP system.

Nodose ganglia sensory neurons: These concentrations of cell bodies (ganglia) have been identified in the necks of young rats and processed by standard procedures.

Merckel cell line: This cell line has been acquired from Dr Tapas Das Gupta, Specialised Cancer Centre, University of Illinois, Chicago, USA.

Neuromast cells: Lateral line cells of fish and amphibia are potential sources for mechanosensory cells. Amphibians, such as the African Clawed Toad (Xenopus laevis), possess lateral line "stitches" arranged as ring around each eye. A dissection procedure was developed to remove the ring of stitches intact, complete with underlying nerve supply. The tissue was further processed by standard procedures in attempts to produce dissociated cell cultures. Preliminary investigations have also been conducted to assess the possibility of producing dissociated cell cultures from the lateral line of trout.

Long term maintenance of active neuronal cultures General tissue culture. Contamination of cultures by bacteria, yeast and/or fungi represents a considerable problem for the long-term maintenance of cells. This is exacerbated by the need to omit antibiotics from the culture medium (see following sections).

The introduction of the use of Flexiperm tissue culture rings has resulted in a large reduction in the volume of medium required for culture maintenance. This is particularly important when expensive medium supplements are required (e.g. nerve growth factor, NGF). It has also produced an improvement in culture quality and longevity due to reduced dilution of soluble trophic factors, secreted by glial cells or the neurons themselves.

Effects of substrate pre-treatment: many cultures of spinal cord and hippocampal neurons successfully achieved 4-week maturity under a variety of different substrate and media combinations. Neuronal attachment was best facilitated by the pre-treatment of MMEP'S with PEI, whether the surface has been flamed or not. Although P-D-L and P-L-L coating alone does not result in long-term attachment of hippocampal and spinal cord cells to the polysiloxane resin the use of these factors following flaming of the MMEP plate produces good cell adhesion. In conclusion, methods have been developed to ensure good attachment of neuronal cells and processes using PEI, P-D-L or P-L-L as the attachment factor.

Cell culture medium effects: the use of a serum free medium formulation used by Gross (SSM-A) generally resulted in poor cultures. Other formulations were investigated including the use of glial cell conditioned medium and top-grade foetal bovine serum. Although a few instances of electrophysiological activity were recorded the cell cultures produced were generally of a very poor quality.

Refinements made to the production of active neuronal cell cultures as follows:
the critical factor that had prevented neuronal activity was identified as the use of antibiotics in the culture medium. This result has fundamental implications. In addition, a commercial source of a specially formulated serum-free medium for neuronal cells was identified. This is now routinely used.

Investigations have determined the exact requirements for the production of an active network of primary neuronal cells. These are as follows:
Good Quality MMEP plates, effective coating of plates, good primary cultures, neurobasal serum free medium, maturation period of at least 3 weeks and no antibiotics in the culture medium.

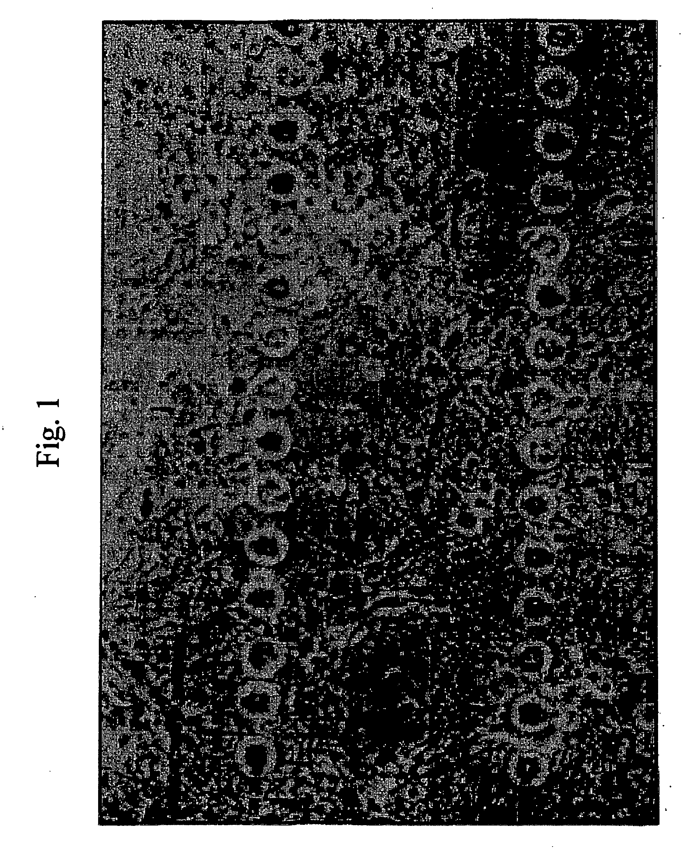

Routine recordings of spontaneous activity were achieved from cultures of hippocampal neurons at 3 - 6 weeks' maturity. A typical hippocampal neuronal network culture is shown in Figure 1. Typically, activity from these cultures can be observed on 75% or more of the 64 channels, with activity ranging from ca. 100(V -1,500(V (peak to peak). In general, it appears that the activity becomes stronger and more copious as the cultures become increasingly mature.

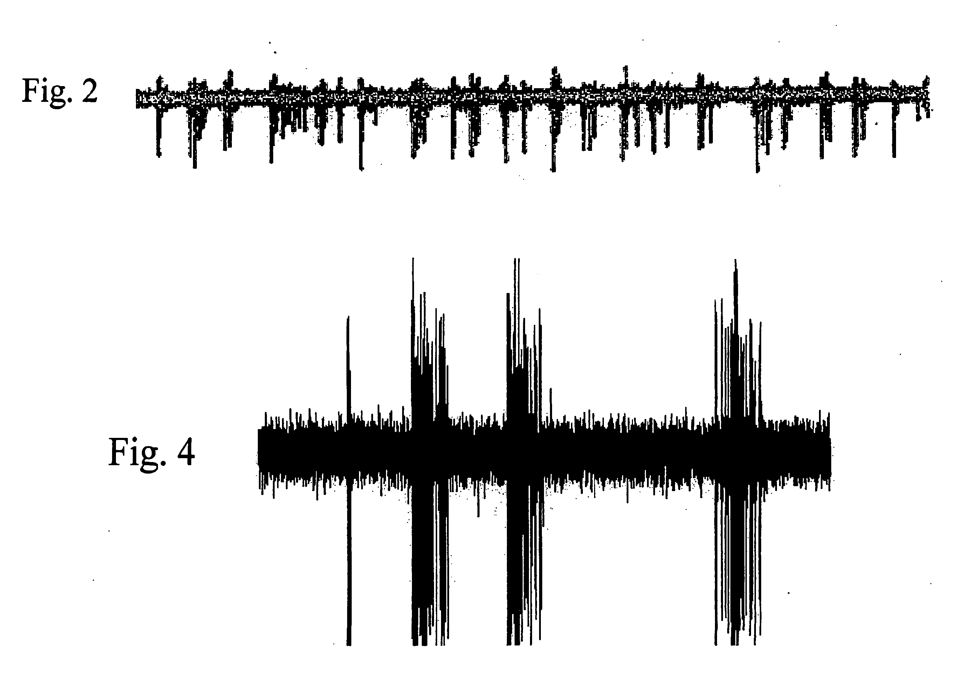

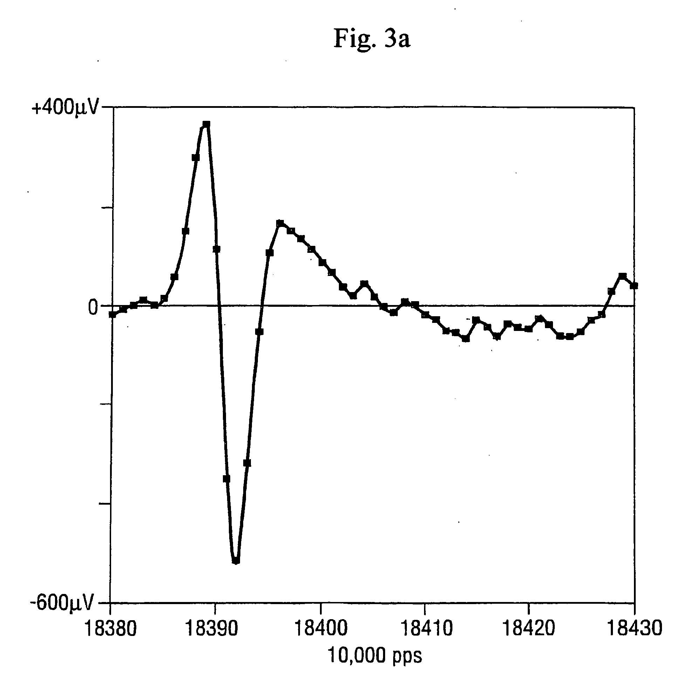

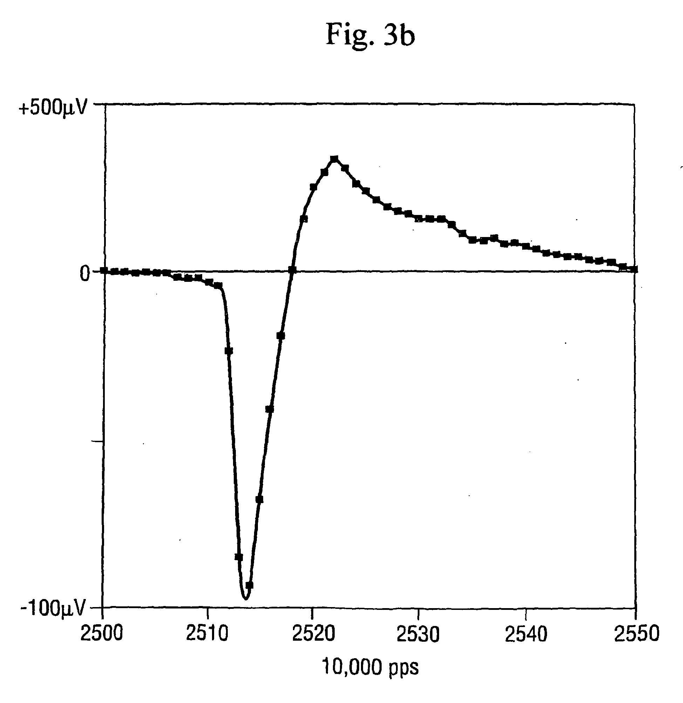

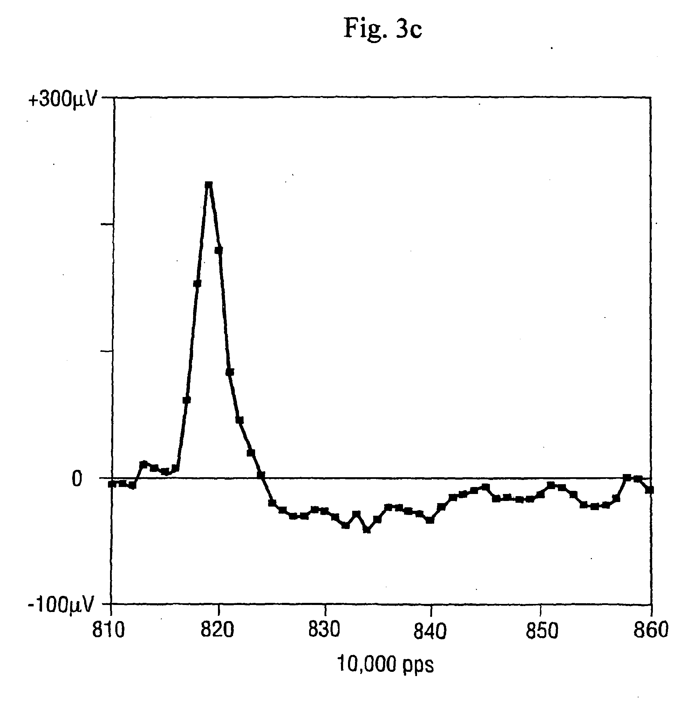

An example trace showing spontaneous activity from a single electrode site in a hippocampal neuronal network is presented in Figure 2. Upon digital analysis, the waveforms appear to present in 3 forms. The first, Figure 3(a), represents a typical, externally recorded action-potential event with an initial positive spike, followed by a larger negative spike and a period of after-hyperpolarisation. The second, Figure 3(b) is the most common waveform, lacks the initial positive spike, and is therefore a pure sodium channel event. The third category of waveform, Figure 3(c), is quite rare, consisting of a positive spike only, probably representative of chloride channel activity.

Primary spinal cord cultures. Neuronal network activity has been routinely observed from rat spinal cord cultures using the MMEP system. A typical trace, highlighting the bursting pattern, is shown in Figure 4. Analysis of the spontaneous activity from spinal cord cultures reveals that they have a similar profile to activity recorded from hippocampal neurons. Typically, activity is present as a series of bursts and individual events can be classified into three categories.

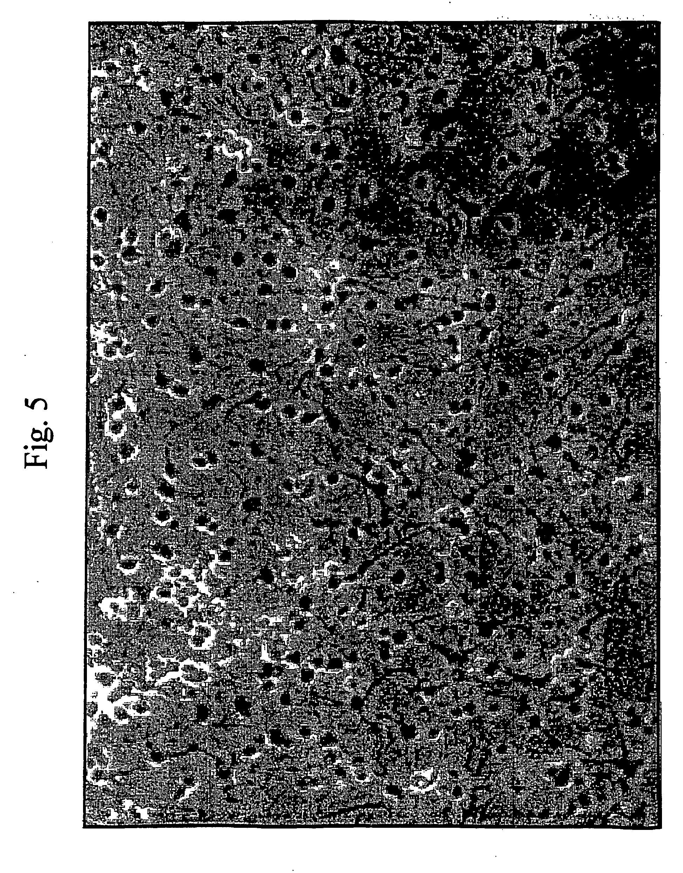

Primary dorsal root ganglia cultures: the dissection of dorsal root ganglia from embryonic rat foetuses and the subsequent culture of dispersed cells were perfected. A representative culture of DRG cells is shown in Figure 5. Analysis of spontaneous activity recorded from mature cultures shows a marked difference from that seen with hippocampal and spinal cord cells.

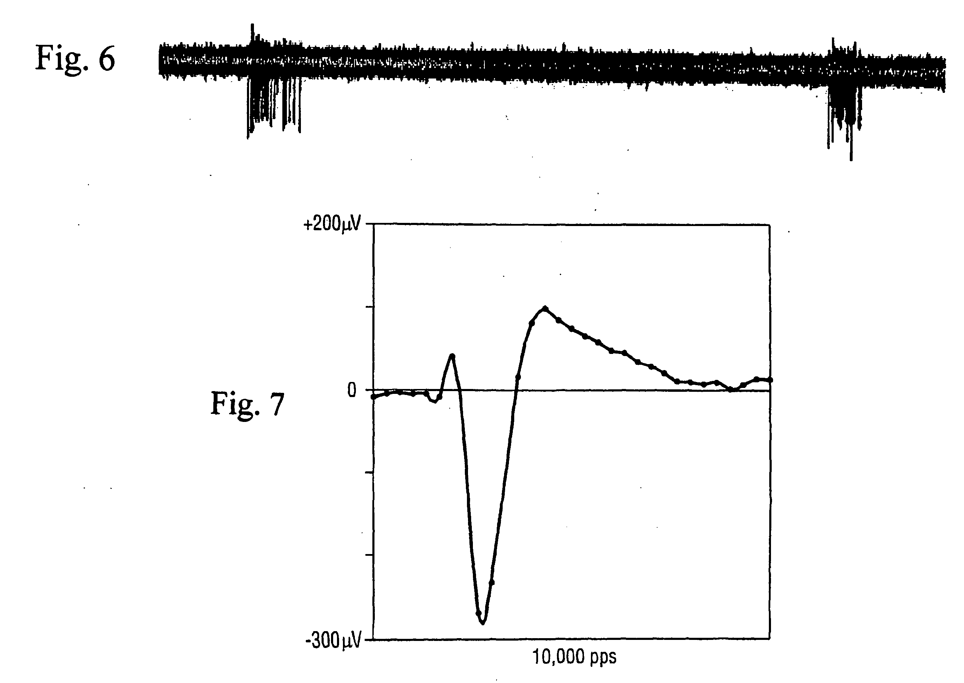

Typical DRG spontaneous activity consists of short bursts of a few spikes, followed by an interval of silence lasting on average 40 seconds (see Figure 6). Occasionally there are intermittent single spikes, or single regular spikes with no bursting. The amplitude of the individual events are generally smaller than that for the hippocampal and spinal cord cultures with the largest event observed to date consisting of a 300(V peak to peak waveform.
Furthermore, the overall activity appears to be less than that for hippocampal and spinal cord cultures. Typically only a very few channels are detected, although on one occasion 50% of the 64 channels showed activity. When analysed, the predominant waveform present consists of a negative spike with some after-hyperpolarisation (Figure 7) similar to the most common type seen with hippocampal and spinal cord cultures (compare Figure 3(a)).

Effects of antibiotics on neuronal network activity: chronic administration of antibiotics to neuronal cell cultures. The chronic effect of antibiotics on neuronal network activity was investigated. Experiments were performed on hippocampal cultures grown in parallel, from the same cell preparation, in the presence or absence of penicillin-streptomycin or gentamycin at the recommended doses. Cell cultures were grown over a 4-week period and analysis of corresponding activity revealed that the addition of antibiotics caused an almost complete cessation of activity. Those few channels that exhibited any activity were very weak (<100 (V peak-to-peak) and often short lived. In contrast, those cultures grown in the absence of antibiotics regularly exhibited activity in excess of 75% of all channels, with some peak-to-peak amplitudes in excess of 1mV, although 300-500 (V was more usual).

In conclusion, it became apparent to the inventors that the production of active neuronal networks required an absence of antibiotics in the culture medium. All techniques were adjusted accordingly. It is important to note that although antibiotics are used routinely in tissue culture applications, there is very little documented evidence that these compounds have any detrimental effect on cell growth. It was decided, therefore, to investigate further the effects antibiotic compounds have on neuronal network activity.

Acute administration of antibiotic compounds to neuronal cell cultures: the majority of experiments involved the investigation of acute addition of a range of antibiotics to hippocampal cultures grown in antibiotic-free Neurobasal medium for 4 weeks. In each case MMEP's were screened for activity and a strong, reliable channel was selected for experimentation. Each antibiotic was tested on at least 3 individual cultures and the antibiotic addition was started at the concentration used routinely in tissue culture studies.

As controls, the anti-fungal agents Nystatin and Amphotericin B (Fungizone), and the anti-mycoplasma (PPLO) agent Tylosin were also tested. In addition, the agent in which the antibiotic compound was dissolved (usually deionised water or 0.9% sodium chloride), as well as normal medium, were also tested for any effects on neuronal activity.

A list of the all the agents tested for the effects of acute addition to hippocampal cultures is presented in Table 1. Particular interest was paid to the effects of penicillin G, streptomycin and penicillin/streptomycin mixtures. These are routinely used in tissue culture medium and therefore any possible toxic effects are particularly relevant.

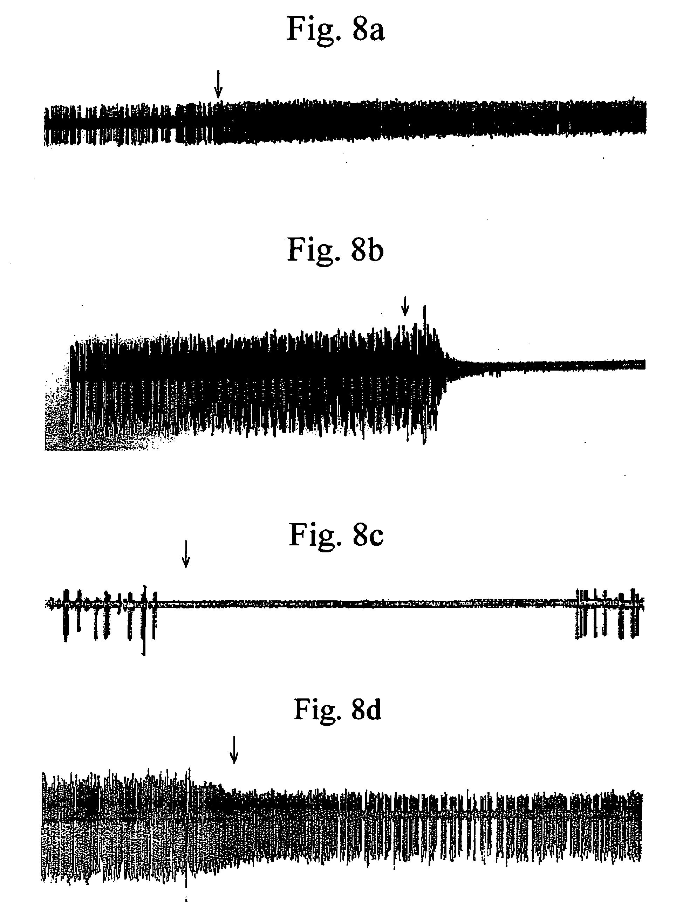

Penicillin G. The addition of 10 µl penicillin G caused an immediate increase in the firing rate of the channel being monitored, with a small associated decrease in the amplitude (see Figure 8 (a)). Subsequent further 10µl additions up to 100µl at approximate 10 second intervals induced a further decrease in the amplitude with eventual slowing of the firing rate.

Streptomycin. Addition of 10µl of streptomycin to an active culture of neuronal cells caused an immediate cessation of firing. If a particularly strong (>500(V) channel was under investigation, the activity would usually begin to recover, without a change of medium, after a silence of approximately 30 seconds. The normal activity was, thereafter, resumed within a few minutes. Further sequential additions of 10µl aliquots of Streptomycin once again caused immediate interruption of activity, which recovered spontaneously after longer time intervals (see Figure 8 (b) ).

If a channel with weak activity (< 500µV) was under study, a single 10µl addition of Streptomycin was usually sufficient to inhibit activity for several minutes, or until a medium change was performed.

In all cases changing the culture to the antibiotic-free formulation reversed the effect. Furthermore, equivalent additions of medium or 0.9% sodium chloride produced no effect in these cultures.

Penicillin G / Streptomycin mix. Further experiments, using standard mixtures of penicillin and streptomycin, were performed to establish a threshold value for inhibition of activity. The concentration for the "all-or-nothing" response lies very close to the normal concentration used in tissue culture medium, a single 10µl addition to a final concentration of 100U/0.1mg/ml being sufficient to inhibit activity (see Figure 8(c)). A final concentration 80-90% of the normal concentration was usually ineffective at inhibiting activity. Very strong channels of activity were again more resistant to inhibition and could recover activity spontaneously.

Gentamicin. The antibiotic gentamicin is also commonly used in tissue culture applications. Addition of this antibiotic, to levels recommended by the supplier, results in a cessation of neuronal activity. This inhibition is reversed by a complete medium change within the MMEP culture apparatus.

Other aminoglycoside antibiotics. Streptomycin belongs to the aminoglycoside group of antibiotics and it was therefore interesting to investigate whether other structurally similar compounds elicit a similar response. A number of aminoglycoside antibiotics were tested and the pattern of inhibition was very similar to that seen for streptomycin addition. Kanamycin was the only exception, with sequential additions of 10µl aliquots, up to 10 times the normal concentration, generally insufficient to inhibit spontaneous activity (see Figure 8(d)). Some minor modification in the firing pattern was, however, occasionally observed.

Other compounds tested. Polymyxin 'B' also caused inhibition of activity, whereas chlortetracycline induced a modification somewhat similar to penicillin G.

No inhibition of activity was seen with the anti-fungal agents amphotericin B and Nystatin, the anti-PPLO (mycoplasma) agent Tylosin, or any of the control additions up to 10 times the usual volume.

The poor frequency of activity detection seen when cultures were grown for several weeks in the presence of Penicillin-Streptomycin or Gentamicin indicates a toxic effect of these compounds after long-term presence. Nouhnejad & Salehian [NOUHNEJAD P, SALEHIAN P (1989) Toxicity and mechanism of action of aminoglycoside antibiotics (gentamycin and amikacin) at the level of neural membranes. Asia Pacific Journal of Pharmacology. 4, 227-231.] It has previously been shown that high doses of gentamicin can cause major structural changes in neuronal cytoarchitecture when administered chronically to guinea pigs.

The fact that some channels (especially those with strong activity) recommenced firing in the acute presence of Streptomycin, without the need to change the chamber medium, suggests that short-term channel blocking effects can be overcome in certain circumstances. Thus also suggests an alternative mechanism for long-term toxic effects.
It is interesting that Penicillin on its own appeared to induce an initial acceleration of the firing rate, with an accompanied reduction in spike amplitude, at up to 10 times the recommended concentration. This is most likely due to the difference in chemical structure of Penicillin related to its mode of action in interfering with the final stage of the synthesis of the bacterial cell wall. Aminoglycoside antibiotics, on the other hand, interfere with bacterial protein synthesis at the level of the ribosomes.

Also of interest is the reduced effectiveness of Kanamycin when compared with other antibiotics of aminoglycoside structure, and similar traditional mode of action. The concentration tested is equivalent to that of the other aminoglcosides used, so the explanation most likely lies in small structural differences making Kanamycin less effective as a channel blocker.

The mode of action of Polymixin 'B' is to bind to the bacterial cytoplasmic membrane and interfere with the permeability of the cell, thus suggesting its' apparent channel blocking activity. Tetracycline functions in a similar manner to the aminoglycosides, by blocking binding of RNA to the 30S subunit of ribosomes, thus inhibiting protein synthesis. It also appears to have a modulatory role on neural activity, without a blocking effect, although the concentration recommended and tested was 0.1-1 times that of the aminoglycosides.
Neuronal network response to pressure stimuli.

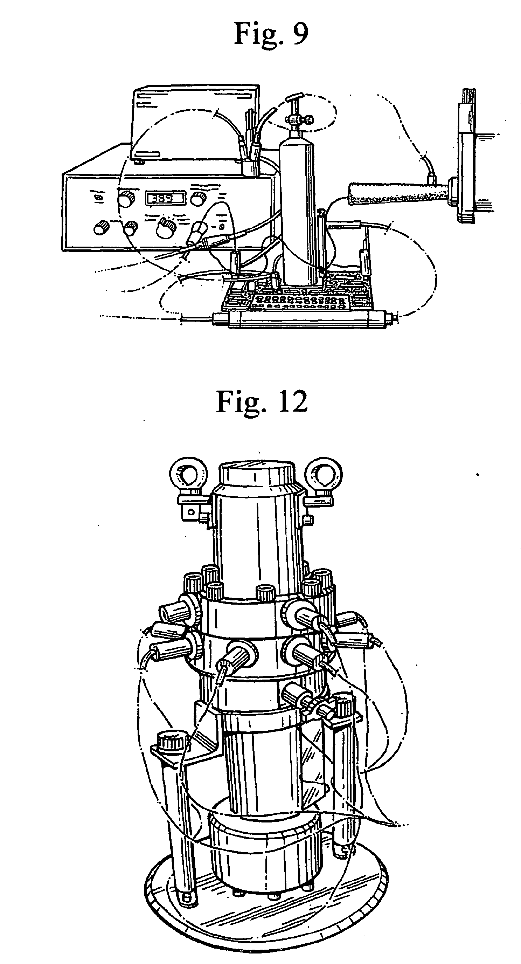

Modifications to the MMEP apparatus. Minor modifications were made to the culture apparatus to enable preliminary pressure response experiments to be performed. In brief, this modification consisted of a 50 ml syringe barrel connected and sealed to the stainless-steel culture chamber (see Figure 9). A further modification has been developed in which a membrane is introduced above the cells to allow the transmission of a physical effect without a damping effect. A lightly weighted syringe barrel is released from various heights above the membrane.

Response of hippocampal cells to static pressure. Using the syringe barrel modification of the MMEP system, the effect of a 2 atmospheres increase in static pressure on hippocampal cell activity was investigated. It was observed that the application of pressure did not result in any modification of spontaneous activity. This result is not unexpected, as it is unlikely that hippocampal cells possess an inherent pressure sensing system.

Response of Dorsal Root Ganglia cells to static pressure. The pattern of spontaneous activity in DRG neurons was markedly different to that observed in hippocampal or spinal cord cultures. Activity occurs in a series of bursts separated by a reasonably constant time delay. Timing of individual burst intervals was made over a 40 min period and a total of 60 bursts occurred with a mean burst interval of 39.8 seconds.

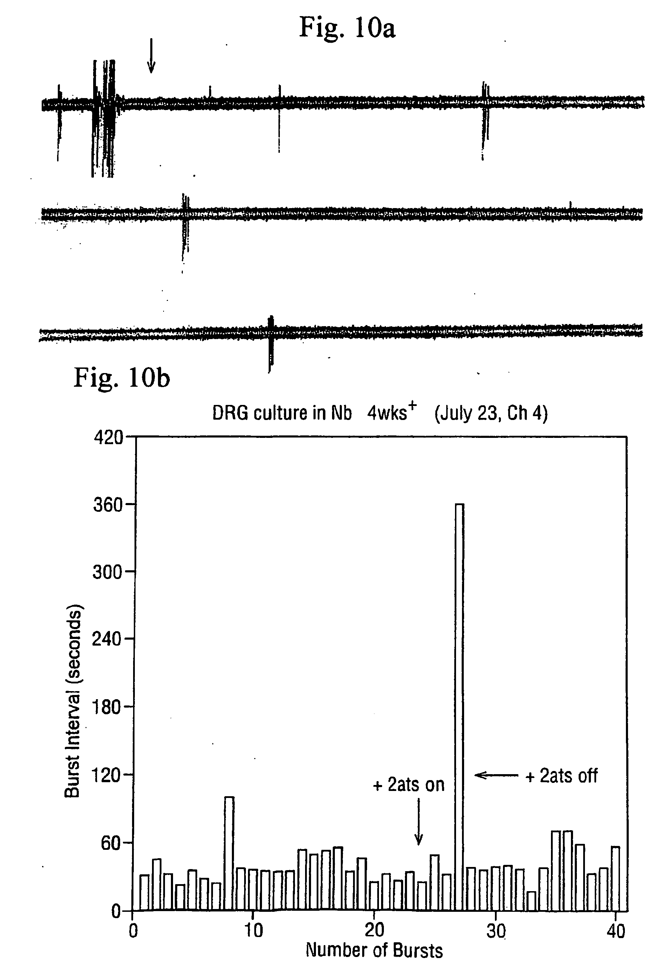

When a pressure of +2 atmospheres was applied from a gas cylinder, via a 50ml syringe barrel clamped to the MMEP chamber, the normal pattern of burst activity was interrupted but resumed upon release of pressure, unless there has been loss of medium from the chamber. This is demonstrated in Figure 10 where no burst occurred after the pressure was applied for a further 4 minutes. At this point the pressure was released and a normal pattern of bursting was resumed. The normal bursting pattern continued for a further 10 minutes (13 bursts).

The demonstration of an inhibitory effect on regular DRG cell bursting with the application of pressure is highly intriguing and suggests a disruption of the "Tensegrity" model, suggested by Ingber [INGBER D E (1997) The architectural basis of cellular mechanotransduction. Annual Rev. Physiol. 59, 575-599.], associated with normal mechanoreceptor function.

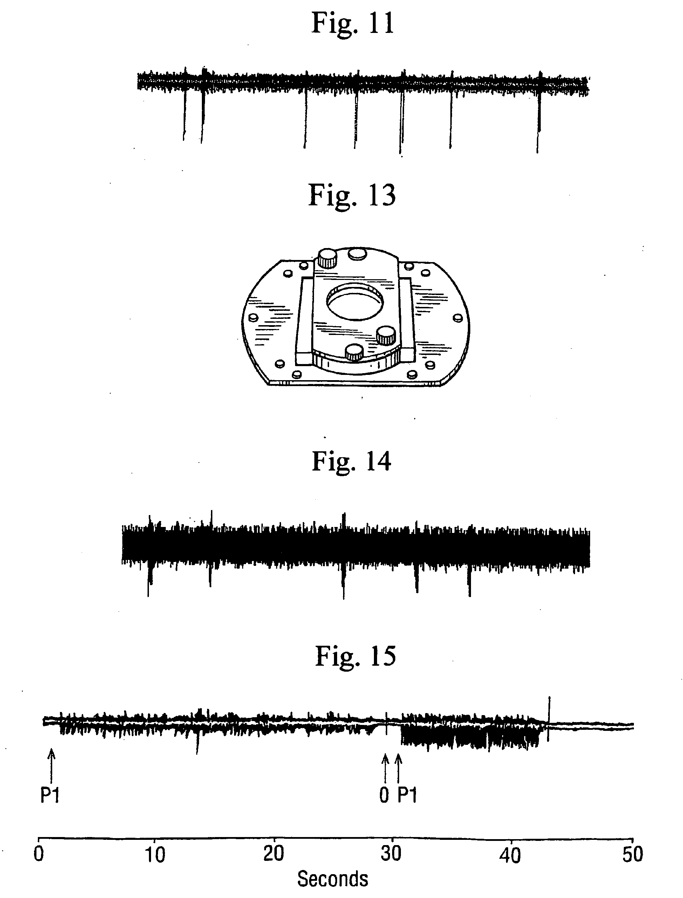

Response of DRG cells to DC pressure. If drops of medium are allowed to fall from the top of the syringe barrel (a distance of 10cm), an audible and visible burst of spikes is stimulated (see Figure 11). These appear to have the same waveform profile as spontaneous activity. There is no stimulus artefact if drops are allowed to fall into an MMEP chamber containing medium, but no cells growing on the plate.

Further analysis of one burst event induced by a falling drop, shows it to be composed of 9 single, well spaced, events occurring over a 160 millisecond time period. Each individual event has a classical waveform pattern thus confirming that they are not artefacts.

Response to pressure induced by a falling weight approach. Using the modification described above the response of a DRG culture to a light weight falling on a membrane lying just above the cells was examined. Initial studies were very promising showing an effect following impact of the weight. Further studies, however, showed that these large artefacts were induced in the presence or absence of cell cultures. Thus, the effect observed was not real.

BioAcoustics Calibration Tube. Subjecting neuronal cell cultures, grown on the MMEP system, to defined acoustic and/or pressure fields requires a unique piece of equipment. The BioAcoustics Calibration (BAC) tube was designed and procured (see Figure 12). The Tube allows the culture of neuronal and bacterial cells under static or acoustic pressure and allows electrical contact to be made at 60 sites within the culture chamber. Additional equipment has been developed to enable the BAC tube to be pressurised. All necessary health and safety checks have been performed on the BAC tube and all ancillary equipment.

Additional developments have been made to enable transmission of acoustic and/or static pressure to neuronal cultures grown on the MMEP system. A smaller base plate has been built that can fit into the chamber of the BAC tube. Furthermore, a water-tight sealed system has been produced ensuring no leakage of culture medium upon experimentation. The system consists of a rubber diaphragm and O-ring that is placed into the stainless steel MMEP culture chamber (see Figure 13). A purpose built steel top plate is bolted onto the culture chamber using the screw holes for the medium re-circulating taps. Neuronal cell cultures on MMEP plates are flooded with medium and the diaphragm put in place, avoiding trapping of air. The O-ring and top plate are bolted down producing a water-tight seal. Using this system spontaneous activity from a foetal rat spinal cord culture present inside the BioAcoustics tube has been recorded (see Figure 14).

The use of the elastomere zebra strip results in a decrease in signal strength. For this reason a Pin-jig arrangement is currently being developed that will enable contact with the 32 edge connectors through spring-loaded gold pins. A miniaturised pre-amplifier board will be added to this arrangement that will fit into the BAC tube. Once the capability of measuring neuronal responses from 64 channels of a MMEP plate has been established, cultures of dorsal root ganglia cells will be subjected to defined acoustic fields and associated pressure responses characterised.

Differentiation studies on cell lines: cell lines provide an almost unlimited supply of material and, therefore, are of enormous benefit to the development of a biological acoustic sensor.
Neuronal cell lines exist in an immature, immortal state to enable their routine culture. The development of a neuronal-like morphology is achieved through the process of differentiation. In this process, cues are given to the cell to instruct it to undergo a pre-determined course of gene expression. These cues are typically, signalling molecules such as polypeptides (e.g: NGF) or chemical compounds (e.g. retinoic acid), that can be added to the cell culture medium.

Dorsal root ganglia cell line ND7/23: the ND7/23 cell line is of particular interest because it was developed from the dorsal root ganglia and, therefore, is potentially pressure responsive. Neurons are produced from the cell line by reducing the serum content (down to 0.5%) and adding dibutryl cAMP and NGF to the cell culture medium as described by Wood *et al*. [WOOD J N, BEVAN S J, COOTE P R, DUNN P M, HARMAR A, HOGAN P, LATCHMAN D S, MORRISON C, ROUGON G, THEVENIAU M, WHEATLEY S. (1990) Novel cell lines display properties of nociceptive sensory neurons. Proc. R. Soc. Lond. B 241, 187-194.].

Transfer of the ND7/23 cells to differentiation medium results in the cessation of multiplication and the development of a classical sensory neurone-like bi-polar morphology within a week in many cells (see Figure 14). A dense network of processes is, however, never achieved and the cells appear morphologically unhealthy within about 2 weeks. This is undoubtedly due to the very low serum content of the differentiation medium. Experiments with a higher serum content (1%) have shown that this is not low enough to prevent the continued vigorous multiplication of the cells.

A culture of differentiated ND7/23 cells, set up on an MMEP plate, was screened for spontaneous activity. Using the syringe barrel arrangement a static pressure equivalent to 2 atmospheres was applied briefly to the culture. On the fifth electrode chosen a 26 second period of activity was recorded coincidental with the pressure application (see Figure 15). The pressure pulse was repeated within a few minutes and resulted in a similar period of neuronal activity, although of greater amplitude and lesser duration. A digitised sample was taken and showed the activity present as a series of negative spikes of - 60µV followed by a short period of hyper-polarisation.

Further pressure application failed to elicit a response and microscopic analysis of the culture revealed that the cells had become detached from the MMEP plate.

P19 cell line: studies on the P19 cell line have indicated that neuronal cells produced are electrically active [MACPHERSON P A, JONES S, PAWSON P A, MARSHALL K C, MCBURNEY M W. (1997) P19 cells differentiate into glutamatergic and glutamate-responsive neurons in vitro. Neuroscience. 80, 487-499.]. The P19 cell line is a mouse teratocarcinoma cell and the neuronal cells are believed to originate from the neocortex. It is unlikely that such cells will possess inherent pressure sensing capabilities, however, the availability of an electrically active cell line would be of enormous benefit to the development of a neuronal acoustic sensor.

P19 cells are embryonic in nature and, therefore, have the potential to differentiate into a number of different phenotypes under the influence of specific factors. Addition of DMSO into the cell culture medium produces muscle cells, whereas retinoic acid supplementation results in the production of neuronal-like cells [JONES-VILLENEUVE E M, MCBURNEY M W, ROGERS K A, KALNINS, V 1. (1982) Retinoic acid induces embryonal carcinoma cells to differentiate into neurons and glial cells. J. Cell Biol. 94, 253-262. MCBURNEY M W, JONES-VILLENEUVE E M, EDWARDS M K, ANDERSON P J. (1982) Control of muscle and neuronal differentiation in a cultured embryonal carcinoma cell line. Nature 299, 165-167.]. Moreover, maintenance of P19 cells in retinoic acid supplemented medium for 28 days produces a dense network of neural processes.

Differentiation experiments have been performed on the P19 cell line using retinoic acid. In initial experiments it was observed that the P19 cells respond to retinoic acid and adopt neuronal-like morphology. However, only 60-70% of the cells responded to the stimulus, such that over an extended period the non-neuronal cells proliferate vigorously and kill the neuronal cells. The use of serum-free medium has overcome this problem but cultures appear unhealthy after a period of 2 weeks.

Rat phaeochromocytoma (PC-12) cells: although networks of fully differentiated, apparently healthy, PC-12 cultures have been produced on MMEP plates no electrical activity has been detected. It has yet to be documented in the literature whether PC-12 cells are electrically active or not. In the light of experiments on the effects of antibiotics on neuronal activity this matter has yet to be resolved as these experiments were performed with a penicillin-streptomycin antibiotic mixture present in the cell culture medium.

**Table 1: List of antimicrobial agents tested for effects of acute addition to hippocampal cultures**

| **Antimicrobial** | **Class of Agent** | **Company** | **Cat No** | **Concentration/ml** |
|---|---|---|---|---|
| Penicillin G | HYDRABAMINE | SIGMA | P-3032 | 100U |
| Streptomycin | AMINOGLYCOSIDE | SIGMA | S-0890 | 0.1 mg |
| Pen-Strep | MIXED | SIGMA | P-4458 | 50U/0.05mg |
| Pen-Strep | MIXED | SIGMA | P-4333 | 100U/0.1mg |
| Gentamicin | AMINOGLYCOSIDE | SIGMA | G-1397 | 0.05mg |
| Geneticin | AMINOGLYCOSIDE | SIGMA | G-7034 | 0.05mg |
| Kanamycin | AMINOGLYCOSIDE | GIBCO | 15160/021 | 0.1mg |
| Neomycin | AMINOGLYCOSIDE | GIBCO | 15310/022 | 0.1mg |
| Lincomycin | AMINOGLYCOSIDE | GIBCO | 25600/016 | 0.05mg |
| Tetracycline | CYCLIC AMINE | GIBCO | 15280/019 | 0.01mg |
| Polymixin 'B' | DAB-AMINE | GIBCO | 15350/010 | 100U |
| Nystatin | POLYENE | GIBCO | 15340/029 | 100U |
| Amphotericin B | POLYENE | SIGMA | A-2942 | 0.25µg |
| Tylosin | MACROLIDE | GIBCO | 15220/023 | 0.01 mg |

## Claims

1. The use of an apparatus comprising living neuronal cells contacting a substrate, wherein said substrate comprises a plurality of electrodes capable of detecting the electrical activity of said cells, for sensing an antibiotic, **characterised in that** said antibiotic comprises an aminoglycoside, tetracycline, lincomycin or polymyxin 'B'.

2. Use according to claim 1, wherein the aminoglycoside is selected from the group consisting of streptomycin, gentamycin, GENETICIN^{®} and neomycin.

3. Use according to claim 1 or claim 2, wherein said neuronal cells are hippocampal cells.

4. Use according to any preceding claim, wherein the antibiotic is a mixture additionally comprising penicillin G.

## Patentansprüche

1. Verwendung einer Vorrichtung, die lebende neuronale Zellen enthält, die mit einem Substrat in Kontakt stehen, wobei das Substrat mehrere Elektroden aufweist, mit denen die elektrische Aktivität der Zellen erfasst werden kann, zum Nachweis eines Antibiotikums, **dadurch gekennzeichnet, dass** das Antibiotikum ein Aminoglycosid, ein Tetracyclin, Lincomycin oder Polymyxin 'B' ist.

2. Verwendung nach Anspruch 1, wobei das Aminoglycosid aus der Gruppe ausgewählt ist, die aus Streptomycin, Gentamycin, GENETICIN^{®} und Neomycin besteht.

3. Verwendung nach Anspruch 1 oder 2, wobei die neuronalen Zellen Hippocampus-Zellen sind.

4. Verwendung nach seinem der vorhergehenden Ansprüche, wobei das Antibiotikum ein Gemisch ist, das zusätzlich Penicillin G enthält.

## Revendications

1. Utilisation d'un appareil comprenant des cellules neuronales vivantes en contact avec un substrat, où ledit substrat comprend une pluralité d'électrodes capables de détecter l'activité électrique desdites cellules, pour détecter un antibiotique, **caractérisée en ce que** ledit antibiotique comprend un aminoglycoside, de la tétracycline, de la lincomycine ou de la polymyxine « B ».

2. Utilisation selon la revendication 1 où l'aminoglycoside est choisi dans le groupe consistant en la streptomycine, la gentamycine, la GENETICINE^{®} et la néomycine,

3. Utilisation selon la revendication 1 ou la revendication 2 où lesdites cellules neuronales sont des cellules hippocampiques.

4. Utilisation selon l'une quelconque des revendications précédentes où l'antibiotique est un mélange comprenant en outre de la pénicilline G.
